## Europäisches Patentamt
## European Patent Office
## Office européen des brevets

(19)

(11) Numéro de publication: **0 172 068**

**A1**

(12)

# DEMANDE DE BREVET EUROPEEN

(21) Numéro de dépôt: **85401415.6**

(22) Date de dépôt: **11.07.85**

(51) Int. Cl.⁴: **C 01 B 33/28**
**B 01 J 29/28, C 07 C 7/13**
**C 07 C 1/20**

(30) Priorité: **20.07.84 FR 8411521**

(43) Date de publication de la demande:
**19.02.86 Bulletin 86/8**

(84) Etats contractants désignés:
**BE DE FR GB IT NL**

(71) Demandeur: **COMPAGNIE FRANCAISE DE RAFFINAGE**
Société anonyme dite:
**5, rue Michel-Ange**
**F-75781 Paris Cedex 16(FR)**

(72) Inventeur: **Guth, Jean-Louis**
**59, rue Bellevue**
**F-68350 Brunstatt(FR)**

(72) Inventeur: **Kessler, Henri**
**17, rue de la Forêt**
**F-68270 Wittenheim(FR)**

(72) Inventeur: **Bourgogne, Michel**
**18, rue Albert Dubosc**
**F-76310 Sainte Adresse(FR)**

(72) Inventeur: **Wey, Raymond**
**29, rue Georges Sand**
**F-68200 Mulhouse(FR)**

(72) Inventeur: **Szabo, Georges**
**38, rue de Normandie**
**F-76290 Montvilliers(FR)**

(74) Mandataire: **Brot, Philippe et al,**
**CABINET BROT et JOLLY 83, rue d'Amsterdam**
**F-75008 Paris(FR)**

(54) **Nouveau procédé de synthèse de zéolites du type aluminosilicate, produits obtenus par ce procédé et utilisation de ces produits.**

(57) L'invention concerne un procédé de synthèse de zéolites.

Ce procédé consiste à former un milieu réactionnel comprenant notamment de l'eau, une source de silice, une source d'alumine, une source d'un agent structurant pouvant fournir des cations choisis dans le groupe constitué par les cations tétrapropylammonium (TPA⁺), tripropylammonium (TRIPA⁺), dipropylammonium (DIPA⁺) et tétrapropylphosphonium (TPP⁺), et des anions fluorure F⁻, le pH du milieu étant inférieur à 10, à chauffer ledit milieu réactionnel à une température comprise entre 60 et 230°C, cette seconde étape conduisant à l'obtention d'un solide cristallisé qui est séparé, et à chauffer ledit solide à une température supérieure à 450° C.

EP 0 172 068 A1

Croydon Printing Company Ltd.

## Nouveau procédé de synthèse de zéolites du type aluminosilicate, produits obtenus par ce procédé et utilisation de ces produits.

La présente invention concerne un nouveau procédé de synthèse de zéolites du type aluminosilicate, les produits obtenus par ce procédé, ainsi que leurs applications en adsorption et en catalyse.

La composition des zéolites du type aluminosilicate est généralement représentée par la formule brute $M_{2/n}O; Al_2O_3; xSiO_2$ à l'état déshydraté et calciné; M représente un élément électropositif de valence n, tel qu'un élément alcalin ou alcalino-terreux; x peut varier de 2 à théoriquement l'infini, auquel cas la zéolite n'est plus un aluminosilicate, mais une silice.

Les zéolites sont cristallisées. Leurs structures sont constituées par un assemblage de tétraèdres $SiO_4$ et $AlO_4$ formant une charpente tridimensionnelle par la mise en commun des atomes d'oxygène. Dans les cavités et pores de dimensions moléculaires de la charpente se logent les cations compensant le déficit de charge dû à la présence de l'aluminium trivalent dans les tétraèdres.

Chaque type de zéolite possède une structure poreuse distincte. La variation des dimensions et formes des pores, d'un type à l'autre, entraîne des changements des propriétés adsorbantes. Seules les molécules de certaines dimensions et formes sont capables d'entrer dans les pores d'une zéolite particulière. La composition chimique, avec, en particulier, la nature des cations de compensation échangeables, est également un facteur important intervenant dans la sélectivité de l'adsorption et surtout dans les propriétés catalytiques de ces produits.

En raison de ces caractéristiques remarquables, les zéolites conviennent tout particulièrement à la purification de gaz ou de liquides et à la séparation d'hydrocarbures par adsorption sélective. De même, elles sont utilisées comme catalyseur ou support de catalyseur, notamment dans le craquage, le reformage et la modification d'hydrocarbures, ainsi que dans l'élaboration de nombreuses molécules.

Bien que de nombreuses zéolites du type aluminosilicate existent dans la nature, la recherche de produits ayant des propriétés nouvelles, a conduit au cours de ces dernières années à la synthèse d'une grande variété de ces aluminosilicates. On désigne généralement ces zéolites par un symbole, par exemple zéolite A (Brevet US N° 2 882 243), zéolite X (Brevet US N° 2 882 244), zéolite Y (Brevet US N° 3 130 007), zéolite L (Brevet français N° 1 224 154), zéolite T (Brevet français N° 1 223 775), zéolite ZSM-5 (Brevet US N° 3 702 886), zéolite ZSM 12 (Brevet US N° 3 832 449), zéolite ZSM 34 (Brevet US N° 4 086 186); zéolite ZSM 48 (Brevet européen N° 0 015 132).

De manière typique, les zéolites sont préparées par cristallisation hydrothermale de mélanges réactionnels contenant des sources d'oxydes de métaux alcalins ou alcalino-terreux, de silice, d'alumine et, dans certains cas, d'une espèce organique. Les milieux réactionnels sont caractérisés par un pH basique généralement supérieur à 10. On admet actuellement que cette concentration en ions $OH^-$ facilite la cristallisation de la zéolite, en assurant la dissolution des sources de silice et d'alumine et le transfert des espèces solubles ainsi obtenues sur la zéolite en voie de formation. Bien que la présence d'espèces organiques, en particulier des cations dérivés de bases organiques, se traduise généralement par une augmentation du rapport $SiO_2/Al_2O_3$ dans la charpente, on pense actuellement qu'elles interviennent directement dans la formation de la structure de certaines zéolites.

Cette méthode de synthèse des zéolites présente de nombreux inconvénients. En effet, dans un milieu basique, la plupart des zéolites synthétisées sont métastables et on risque l'apparition, au cours de la préparation, de phases solides plus stables, mais non désirées. Cette difficulté ne fait que s'accroître lorsque les quantités préparées augmentent c'est-à-dire lorsqu'on passe de l'échelle du laboratoire à l'échelle industrielle. D'autre part, ces zéolites métastables ne sont obtenues que grâce à une forte sursaturation en espèces actives dans le milieu, ce qui provoque une nucléation rapide et, par conséquent, conduit à des cristaux de zéolites de petite taille. Les dimensions moyennes se situent dans la zone du micromètre. L'obtention de cristaux avec des dimensions de quelques dizaines de micromètres est difficile, donc rare. Or, dans un certain nombre d'applications (échange d'ions, adsorption, catalyse), il serait intéressant de pouvoir travailler avec des cristaux de grande taille, ce qui, par exemple, permettrait d'éviter le conditionnement des zéolites par agglomération avec tous ses inconvénients.

De nombreuses applications, en particulier dans la catalyse acide, nécessitent des zéolites sous une forme protonée et complètement débarrassées de leurs cations de compensation alcalins ou alcalino-terreux introduits lors de la synthèse. On peut y accéder par des procédés d'échange répétés et longs avec des cations $NH_4^+$, suivis de calcinations pour les décomposer en cation $H^+$. Cette étape d'échange d'ions pourrait être éliminée, si on pouvait entièrement remplacer les cations de métaux alcalins par des cations $NH_4^+$ lors de la synthèse. Or ceci n'est pas possible, lorsque le pH dépasse sensiblement 10, les cations $NH_4^+$ étant dans ces conditions transformés en ammoniac. D'autre part, les synthèses effectuées à des pH où le cation $NH_4^+$ est stable sont difficiles et longues, à cause de la faible solubilité des sources de silice et d'alumine à ces bas pH.

La Demanderesse a mis au point un nouveau procédé de synthèse de zéolites, permettant d'éviter les inconvénients des procédés de synthèse déjà connus décrits ci-dessus et conduisant à des zéolites du type ZSM 5 possédant des propriétés particulières.

Le but de la présente invention est donc la mise au point d'un procédé perfectionné de synthèse de zéolites.

A cet effet, l'invention a pour objet un procédé de synthèse de zéolites, ledit procédé consistant:

a) dans une première étape, à former un milieu réactionnel comprenant notamment de l'eau, une source de silice, une source d'alumine, une source d'un agent structurant pouvant fournir des cations choisis dans le groupe constitué par les cations tétrapropylammonium (TPA$^+$), tripropylammonium (TRIPA$^+$), dipropylammonium (DIPA$^+$) et tétrapropylphosphonium (TPP$^+$),

b) dans une deuxième étape, à chauffer le milieu réactionnel formé dans l'étape a) à une température comprise entre 60 et 230° C et, de préférence, entre 80 et 210° C, cette seconde étape conduisant à l'obtention d'un solide cristallisé, qui est séparé,

c) dans une troisième étape, à chauffer le solide obtenu dans l'étape b) à une température supérieure à 450° C, ledit procédé étant caractérisé en ce que le milieu réactionnel de l'étape a) contient en outre des anions fluorure F$^-$ et en ce que le pH dudit milieu est inférieur à 10.

L'invention a également pour objet les zéolites préparées par ledit procédé et des applications desdites zéolites.

Le pH inférieur à 10 du milieu réactionnel peut être obtenu soit directement à partir de l'un ou plusieurs des produits composant le milieu réactionnel, soit par l'ajout audit milieu d'un acide, d'une base, d'un sel acide, d'un sel basique ou d'un mélange tampon complémentaire.

Les anions fluorure F⁻ peuvent être introduits dans le milieu réactionnel sous forme de fluorures, comme le fluorure de sodium $NaF$, le fluorure d'ammonium $NH_4F$, le fluorure acide d'ammonium $NH_4 H F_2$, le fluorure de tétrapropylammonium $(C_3H_7)_4NF$, le fluorure de tripropylammonium $(C_3H_7)_3NHF$, le fluorure de dipropylammonium $(C_3H_7)_2 NH_2F$, le fluorure de tétrapropylphosphonium $(C_3H_7)_4PF$, ou de composés hydrolysables pouvant libérer des anions fluorure dans l'eau, comme le fluorure de silicium $SiF_4$ ou le fluosilicate de sodium $Na_2SiF_6$.

Le fluorure d'ammonium ou le fluorure d'ammonium acide sont des sels préférés, car ils permettent l'obtention d'une zéolite apparentée à la ZSM 5, facile à transformer en sa forme protonée sans qu'il y ait lieu de procéder à des réactions d'échange d'ions.

De nombreuses sources de silice peuvent être utilisées dans la formation du milieu réactionnel. On peut citer, par exemple:

- les silices sous forme d'hydrogels, d'aérogels, de suspensions colloïdales,

- les silices résultant de la précipitation de solutions de silicates solubles, ou de l'hydrolyse d'esters siliciques comme l'ester tétraéthylique de l'acide monoorthosilicique $Si (OC_2H_5)_4$, ou de complexes comme le fluosilicate de sodium $Na_2SiF_6$ ou d'ammonium $(NH_4)_2SiF_6$,

- les silices préparées par des traitements d'extractions et d'activation de composés cristallisés naturels ou synthétiques, comme les silicates d'aluminium, les aluminosilicates, les argiles, etc.

Les silices utilisées peuvent être divisées ou agglomérées.

Parmi les sources d'alumine utilisables on peut citer les sels d'aluminium (sulfate, nitrate, chlorure, fluorure, acétate par exemple), les hydroxydes et oxydes d'aluminium, les aluminates, les esters comme l'ester tripropylique de l'acide monoorthoaluminique $Al(OC_3H_7)_3$.

Au lieu de partir de sources séparées d'alumine et de silice, on peut aussi utiliser des sources où les deux oxydes sont combinés, comme, par exemple, les gels de silice-alumine amorphes, les aluminosilicates cristallisés, parmi lesquels on peut citer les argiles et les zéolites.

Les sources de silice et d'alumine peuvent être engagées sous forme soluble ou solide, mais également sous forme d'agglomérats comme des extrudés ou des pastilles. Ce dernier conditionnement convient bien aux sources à base de zéolites brutes ou modifiées déjà agglomérées, qui peuvent ainsi être transformées, selon le nouveau procédé, en zéolites préformées.

Les cations tétrapropylammonium ($TPA^+$) ou tétrapropylphosphonium ($TPP^+$) qui sont les agents structurants sont ajoutés de préférence sous forme de leurs sels. Mais ils peuvent également être engendrés in situ à partir de tripropylamine ou de tripropylphosphine et d'un halogénure de propyle. Les cations tripropylammonium et dipropylammonium sont ajoutés sous la forme de leurs sels ou de leurs bases (tripropylamine ou dipropylamine). Dans ce dernier cas il faut ajouter un acide complémentaire pour ajuster le pH au-dessous de 10.

Les acides ou sels acides, les bases ou sels basiques ajoutés éventuellement en complément pour amener le pH du milieu réactionnel à la valeur désirée peuvent être choisis parmi les acides courants, comme l'acide fluorhydrique HF,

l'acide chlorhydrique HCl, l'acide nitrique $HNO_3$, l'acide sulfurique $H_2SO_4$, l'acide acétique $CH_3COOH$, ou les sels acides comme le fluorure acide d'ammonium $NH_4HF_2$, le fluorure acide de potassium $KHF_2$, le sulfate acide de sodium $NaHSO_4$, le sulfate acide de potassium $KHSO_4$, le phosphate acide de sodium $NaH_2PO_4$ et les bases courantes comme l'ammoniaque $NH_4OH$, la soude NaOH, la potasse KOH ou les sels basiques courants comme les carbonates acide $NaHCO_3$ ou neutre $Na_2CO_3$ de sodium, l'acétate de sodium $CH_3COONa$, les sulfures neutres $Na_2S$ ou acide NaHS de sodium ou les mélanges tampons comme acide acétique $CH_3COOH$-acétate de sodium $CH_3COONa$, ammoniaque $NH_4OH$- chlorure d'ammonium $NH_4Cl$.

La morphologie, la taille et la cinétique de formation de cristaux de zéolites obtenues selon le procédé de l'invention peuvent être modifiées par l'introduction dans le milieu réactionnel de sels complémentaires comme le chlorure de sodium NaCl, le chlorure de potassium KCl, le chlorure d'ammonium $NH_4Cl$, le sulfate de sodium $Na_2SO_4$ et/ou de cristaux (broyés ou non) de composés solides apparentés aux zéolites préparées par le procédé de l'invention.

La première étape du procédé selon l'invention consiste à chauffer à une température comprise entre 60 et 230° C, de préférence entre 80 et 210° C, un milieu réactionnel défini par les valeurs de pH et les proportions relatives des différents constituants figurant dans le tableau I ci-après:

TABLEAU   I

|  | | Domaine large | Domaine préféré |
|---|---|---|---|
| | pH | 1,5    -    10 | 3      - 8,5 |
| Rapports molaires | $SiO_2/Al_2O_3$ | 8     -    500 | 12     - 400 |
| | $F/SiO_2$ | 0,04   -    4 | 0,06 - 2 |
| | $TPA^+$ ou $TPP^+/SiO_2$ * | 0,04   -    1 | 0,08 - 0,75 |
| | $TRIPA^+/SiO_2$ * | 0,2    -    2 | 0,5   - 1,25 |
| | $DIPA^+/SiO_2$ * | 0,5    -   2,5 | 0,75 - 2 |
| | $H_2O / SiO_2$ | 3      -    200 | 4      - 60 |

* Un seul type de cation structurant est nécessaire. Si le choix se porte sur le $TRIPA^+$ ou le $DIPA^+$, il est particu- lièrement recommandé d'ajouter des cristaux de zéolites, qui serviront de germes, dans les proportions indiquées dans le tableau II.

On peut faire varier la morphologie et la taille des cris- taux et la cinétique de la réaction de cristallisation en faisant varier les paramètres figurant dans le Tableau II ci-après.

TABLEAU  II

|  | Domaine large | Domaine préféré |
|---|---|---|
| Rapport molaire sel complémentaire /SiO$_2$ | 0  -  4 | 0  -  0,5 |
| Rapport pondéral cristaux de zéolite complémentaire / SiO$_2$ | 0,01  -  0,10 | 0,02  -  0,04 |

Le chauffage du milieu réactionnel se fait de préférence dans un autoclave revêtu intérieurement de polytétrafluoroéthylène. Suivant la composition du milieu et la température de chauffage, la durée de celui-ci se situe généralement entre 6 heures et 350 heures. Lorsque la cristallisation est achevée, le solide obtenu est filtré et lavé à l'eau désionisée.

La troisième étape de la préparation consiste à chauffer les cristaux obtenus dans l'étape précédente à une température supérieure à 450°C, de façon à détruire et à chasser les espèces organiques liées à l'agent structurant, présentes dans les pores de la zéolite.

Les produits obtenus par le procédé selon l'invention, apparentés aux zéolites du type ZSM 5, sont caractérisés après calcination, par les formules exprimées en oxydes

$$M_{2/n}O; \ Al_2O_3; \ xSiO_2$$

où x peut varier de 12 à 400 et où M représente les cations de compensation de valence n. Après synthèse et calcination, M représente le proton résultant de la décomposition des cations ($NH_4^+$, $TPA^+$, $TRIPA^+$, $DIPA^+$ ou $TPP^+$) et/ou les cations issus du milieu réactionnel. On peut introduire dans les zéolites, par des méthodes d'échange d'ions bien connues, les éléments du tableau périodique dont les cations peuvent être préparés en milieu aqueux. On citera à titre d'exemple les cations alcalins, alcalino-terreux, les cations de terres rares, $Fe^{II}$, $Fe^{III}$, $Co^{II}$, $Co^{III}$, $Ni^{II}$, $Cu^{II}$, $Zn^{II}$, etc.

L'analyse chimique montre qu'il y a en outre entre 0,02 et 1,2 % pondéral d'élément fluor dans les zéolites après calcination.

L'identification de la zéolite du type aluminosilicate obtenue selon l'invention se fait de manière commode à partir de son diagramme de diffraction des rayons X. Ce diagramme de diffraction peut être obtenu à l'aide d'un diffracto-mètre en utilisant la méthode classique des poudres avec le rayonnement $K\alpha$ du cuivre. A partir de la position des pics de diffraction, représentée par l'angle $2\theta$, on calcule, par la relation de Bragg, les équi-distances réticulaires $d_{hkl}$ caractéristiques de l'échantillon. L'estimation de l'erreur de mesure $\Delta(d_{hkl})$ sur $d_{hkl}$ se calcule, en fonction de l'erreur absolue $\Delta(2\theta)$ affectée à la mesure de $2\theta$, par la relation de Bragg. Cette erreur absolue $\Delta(2\theta)$, égale à $\pm 0,2$, est couramment admise. L'intensité relative $I/Io$, I étant l'intensité d'une raie donnée et Io celle de la raie la plus forte, affectée à chaque valeur de $d_{hkl}$ est estimée à partir de la hauteur du pic de diffraction correspondant.

Le Tableau III ci-après représente le diagramme de diffraction des rayons X caractéristique des zéolites du type aluminosilicate obtenues selon l'invention et calcinées à 550° C. Dans la colonne des $d_{hkl}$ on a représenté les valeurs extrêmes que peuvent prendre les différentes équi-distances réticulaires $d_{hkl}$: les variations observées sont essentiellement liées à la nature des cations de compensation et au rapport Si/Al de la zéolite. Chacune de ces valeurs doit encore être affectée de l'erreur de mesure $\Delta(d_{hkl})$.

On a utilisé dans ce Tableau, pour caractériser l'intensité relative I/Io, une échelle de symboles souvent utilisée:
FF = très fort, F = fort, mF = moyen à fort, m = moyen,
mf = moyen à faible, f = faible, ff = très faible.

## TABLEAU III

| $d_{hkl}$ (Å) $(10^{-10}$ m) | I/Io | $d_{hkl}$ (Å) $(10^{-10}$ m) | I/Io | $d_{hkl}$ (Å) $(10^{-10}$ m) | I/Io |
|---|---|---|---|---|---|
| 11,08 - 11,26 | FF | 4,06 - 4,10 | ff | 2,772 - 2,793 | ff |
| 9,94 - 10,20 | mf | 3,99 - 4,05 | f | 2,725 - 2,749 | ff |
| 9,68 - 9,90 | f | 3,83 - 3,89 | F | 2,677 - 2,697 | ff |
| 8,98 - 9,08 | ff | 3,80 - 3,86 | m | 2,648 - 2,670 | ff |
| 8,00 - 8,09 | ff | 3,74 - 3,78 | mf | 2,605 - 2,619 | ff |
| 7,40 - 7,52 | ff | 3,70 - 3,74 | mf | 2,581 - 2,597 | ff |
| 7,03 - 7,22 | ff | 3,63 - 3,67 | mf | 2,545 - 2,557 | ff |
| 6,64 - 6,84 | f | 3,58 - 3,62 | ff | 2,508 - 2,526 | ff |
| 6,30 - 6,42 | f | 3,46 - 3,50 | ff | 2,479 - 2,501 | ff |
| 5,95 - 6,07 | f | 3,42 - 3,46 | f | 2,407 - 2,419 | ff |
| 5,67 - 5,79 | f | 3,38 - 3,42 | ff | 2,393 - 2,401 | ff |
| 5,54 - 5,61 | f | 3,33 - 3,37 | f | 2,326 - 2,340 | ff |
| 5,32 - 5,42 | ff | 3,29 - 3,33 | ff | 2,314 - 2,332 | ff |
| 5,10 - 5,23 | ff | 3,23 - 3,27 | ff | 2,195 - 2,209 | ff |
| 5,01 - 5,08 | f | 3,16 - 3,20 | ff | 2,104 - 2,120 | ff |
| 4,95 - 5,03 | f | 3,12 - 3,16 | ff | 2,077 - 2,095 | ff |
| 4,84 - 4,93 | ff | 3,08 - 3,12 | ff | 2,070 - 2,084 | ff |
| 4,59 - 4,64 | ff | 3,03 - 3,07 | f | 2,004 - 2,022 | f |
| 4,44 - 4,50 | ff | 2,976-3,020 | f | 1,985 - 2,005 | f |
| 4,34 - 4,40 | f | 2,943-2,962 | f | 1,944 - 1,964 | ff |
| 4,23 - 4,29 | f | 2,855-2,881 | ff | 1,907 - 1,922 | ff |
| | | | | 1,866 - 1,881 | ff |

Les zéolites préparées par le procédé selon l'invention s'apparentent aux zéolites du type ZSM 5, comme le montre le Tableau III.

Les zéolites préparées selon l'invention sont des adsorbants sélectifs. Ainsi, ils permettent la séparation des xylènes par adsorption sélective du para-xylène. Mais leur principal intérêt réside dans leur utilisation comme catalyseurs ou support de catalyseurs, purs ou en mélange, pour des transformations très spécifiques de composés organiques variés, allant des hydrocarbures à des composés contenant, outre du carbone et de l'hydrogène, de l'oxygène, de l'azote, du soufre, etc. A titre d'exemple, on peut citer, pour les hydrocarbures, le craquage, l'hydrocraquage, le réformage, l'oligomérisation des oléfines, l'aromatisation des paraffines et des oléfines, la dismutation d'aromatiques, la transalkylation d'aromatiques comme l'isomérisation des xylènes. On peut également citer la transformation des alcools comme le méthanol en hydrocarbures.

Les exemples qui suivent sont destinés à illustrer l'invention de façon non limitative.

EXEMPLE 1

Cet exemple concerne la préparation de zéolites par le procédé selon l'invention.

Préparation N° 1

On prépare une solution contenant 4 grammes de bromure de tétrapropylammonium (TPA - Br) et 1,26 gramme de fluorure de sodium dans 30 grammes d'eau. On mélange ensuite cette solution avec 0,21 gramme d'hydroxyde d'aluminium Al (OH)$_3$ et 2,2 grammes de silice précipitée en poudre, contenant 16 % en poids d'eau, commercialisée par la Société MERCK. On ajoute ensuite quelques gouttes d'une solution d'acide fluorhydrique à 5 %, jusqu'à l'obtention d'un pH 6. La composition molaire du mélange, ramenée à 1 mole de silice, est:

$$1\ SiO_2;\ 0,05\ Al\ (OH)_3;\ 1\ NaF;\ 0,5\ TPA\text{-}BR;\ 56\ H_2O.$$

Le mélange est chauffé pendant 6 jours à une température de 170° C, dans un autoclave revêtu intérieurement de polytétrafluoroéthylène. Le solide obtenu (2,3 grammes), après filtration et lavage à l'eau, est calciné pendant 2 heures à une température de 550°C. L'analyse par diffraction des rayons X montre qu'il s'agit d'une zéolite caractérisée par le diagramme de diffraction du Tableau III, avec des traces de gibbsite ($Al(OH)_3$). La taille des cristaux de zéolite, de forme prismatique, est voisine de 150 x 30 micromètres. On peut aisément séparer la gibbsite (cristaux de taille inférieure au micron) par décantation dans l'eau. L'analyse chimique de la zéolite ainsi purifiée donne un rapport Si/Al de 75 et une teneur en élément fluor de 0,35 % en poids.

Préparation N° 2

On prépare un mélange ayant la composition molaire suivante:

1 $SiO_2$; 0,2 $AlF_3$; 1 KF; 0,25 TPA-Br; 0,2 KCl; 27,5 $H_2O$;

en utilisant 1/200$^e$ des quantités molaires indiquées.

On utilise la même source de silice que dans l'exemple 1 et du fluorure d'aluminium hydraté $AlF_3$, 3 $H_2O$.

Le mélange est chauffé à une température de 150° C pendant 3 jours 1/2 dans un tube de verre pyrex scellé. Après séparation, filtration, lavage et calcination à une température de 550° C pendant 2 heures, on obtient des cristaux de solide d'une taille de 80 x 60 micromètres. Ce solide contient 1,25 % en poids d'aluminium et 0,75 % en poids de fluor. Son diagramme de diffraction des rayons X correspond à celui du Tableau III.

Préparations N° 3 et N° 4

Dans ces préparations, on utilise les mêmes sources de silice et d'alumine que pour la préparation N° 2. Les compositions molaires des mélanges, ramenées à une mole de silice, figurent dans le Tableau IV ci-après:

TABLEAU IV

| Préparation N° | Silice MERCK | $AlF_3$, $3H_2O$ | TPA-Br | $NH_4F$ | $NH_4HF_2$ | $H_2O$ |
|---|---|---|---|---|---|---|
| 3 | 1 | 0,050 | 0,5 | 0 | 1 | 50 |
| 4 | 1 | 0,066 | 0,5 | 1 | 0 | 33 |

On a utilisé respectivement 0,75 et 0,65 mole de silice dans les préparations 3 et 4.

Les réactions de cristallisation sont effectuées en autoclave, dans un flacon de polytétrafluoroéthylène d'une contenance de un litre.

Les conditions opératoires sont données dans le Tableau V ci-après:

TABLEAU V

| Préparation N° | Température en °C | Durée en jours | pH | |
|---|---|---|---|---|
| | | | Initial | Final |
| 3 | 166 | 6 | 5,5 | 5,5 |
| 4 | 204 | 3 | 7 | 7 |

Les produits solides obtenus sont séparés par filtration, puis lavés avec de l'eau acidulée, afin d'éliminer les éventuelles traces de fluorures complexes d'aluminium et
d'ammonium comme $NH_4AlF_4$ ou $(NH_4)_3AlF_6$. Après calcination
à une température de 550° C, on obtient des zéolites ayant
le diagramme de diffraction des rayons X du Tableau III.

Les autres caractéristiques des produits sont données dans
le Tableau VI ci-après:

TABLEAU VI

| Préparation N° | Taille des cristaux en micro-mètres | Composition après calcination à 550° C et réhydratation | | | |
|---|---|---|---|---|---|
| | | % Al en poids | % F en poids | % Na en poids | Rapport Si/al molaire |
| 3 | 40 x 15 | 0,68 | 0,62 | <0,05 | 61 |
| 4 | 100 x 40 | 1,09 | 0,12 | <0,05 | 40 |

Ces préparations N° 3 et N° 4 montrent qu'il est possible
d'obtenir directement, par le procédé selon l'invention, des
zéolites du type ZSM 5 ne contenant que des traces d'ions
alcalins et ne nécessitant pas de longues opérations d'échanges
d'ions pour aboutir aux formes protonées.

Par un traitement hydrolysant, on peut éliminer pratiquement
de manière totale l'élément fluor contenu dans ces zéolites.
Ainsi, un échantillon de la préparation N° 4 chauffé à une
température de 160° C pendant 6 heures dans un autoclave,
avec une solution d'ammoniaque d'une concentration de 0,15
mole/litre, avec un rapport liquide/solide de 5, contient
après le traitement moins de 0,03 % en poids de fluor.

Préparations N° 5, N° 6 et N° 7

Dans ces préparations, on utilise une même source de silice et d'alumine. On utilise pour cela une silice-alumine amorphe obtenue en traitant le produit TIXOLEX 28 commercialisé par RHONE-POULENC par une solution d'acide nitrique 0,5 N pendant 2 heures, à une température de 25° C, avec un rapport liquide/ solide de 10, le produit étant ensuite séché à l'air. La composition pondérale du TIXOLEX 28 traité est la suivante:

$SiO_2$ : 74,3 %
$Al_2O_3$ : 4,82 %
$Na_2O$ : 1,40 %
$H_2O$ : 19,4 %

Le rapport molaire Si/Al est donc de 13.

Les compositions molaires des mélanges réactionnels, ramenés à 1 mole de silice contenue dans le TIXOLEX 28 traité sont données dans le Tableau VII ci-après:

TABLEAU VII

| Préparation N° | TPA - Br | $NH_4$-F | $NH_4HF_2$ | HF | $H_2O$ |
|---|---|---|---|---|---|
| 5 | 0,5 | 0,5 | 0 | 0 | 8 |
| 6 | 0,5 | 0 | 0,25 | 0 | 8 |
| 7 | 0,5 | 0 | 0,375 | 0,125 | 8 |

Les conditions opératoires sont données dans le Tableau VIII ci-après:

TABLEAU VIII

| Préparation N° | Température en °C | Durée en jours | pH | |
|---|---|---|---|---|
| | | | Initial | Final |
| 5 | 190 | 6 | 8 | 8 |
| 6 | 190 | 6 | 6 | 6 |
| 7 | 190 | 6 | 5,5 | 5 |

Les diagrammes de diffraction des produits obtenus calcinés, semblables à ceux du Tableau III, montrent qu'il s'agit de zéolites du type ZSM 5.

Les autres caractéristiques de ces produits sont données dans le Tableau IX ci-après:

TABLEAU IX

| Préparation N.° | Taille des cristaux en microns | Composition après calcination à 550° C et réhydratation | | |
|---|---|---|---|---|
| | | % Al en poids | % F en poids | Rapport Si/Al molaire |
| 5 | 40 x 25 | 1,42 | 0,11 | 29 |
| 6 | 40 x 18 | 2,12 | 0,20 | 18 |
| 7 | 55 x 12 | 0,97 | 0,25 | 46 |

Préparation N° 8

La préparation N° 8 illustre l'influence de l'addition de cristaux de zéolite broyés au milieu réactionnel, sur la taille des cristaux obtenus par le procédé selon l'invention.

On dispose d'une silice-alumine amorphe dont la composition pondérale est la suivante:

$$SiO_2 \quad : 94,65 \text{ %,}$$
$$Al_2O_3 \quad : \phantom{0}2,5 \phantom{0}\text{ %,}$$
$$H_2O \quad : \phantom{0}2 \phantom{00}\text{ %.}$$

On ajoute 21,6 grammes de cette silice-amorphe à une solution de 0,89 gramme de fluorure d'ammonium $NH_4F$ et 6,38 grammes de bromure de tétrapropylammonium TPA-BR dans 21,5 grammes d'eau. On disperse ensuite dans ce mélange 0,4 gramme de cristaux de zéolite brute de synthèse du type ZSM 5 finement broyés.

Les rapports molaires dans le mélange réactionnel sont les suivants:

$$SiO_2/Al_2O_3 \quad : 65,$$
$$NH_4F/SiO_2 \quad : \phantom{0}0,075,$$
$$TPA\text{-}Br/SiO_2 \quad : \phantom{0}0,075,$$
$$H_2O \phantom{0}/SiO_2 \quad : \phantom{0}4.$$

Le pH du milieu réactionnel est de 7.

Le mélange est chauffé à une température de 170° C pendant 6 jours dans un autoclave de 80 millilitres revêtu intérieurement de polytétrafluoroéthylène.

Après refroidissement de l'autoclave, le solide est séparé par filtration, lavé à l'eau, puis calciné pendant 2 heures à une température de 550° C.

La zéolite du type ZSM 5 ainsi obtenue, possédant le diagramme de diffraction des rayons X du Tableau III, se présente sous la forme d'une poudre fine constituée de cristaux dont les dimensions ne dépassent pas quelques micromètres.

La teneur en aluminium de cette poudre est de 1,01 % en poids et, en fluor, de 0,17 % en poids.

Le rapport atomique Si/Al est de 42.

Préparations N° 9 et N° 10

Les préparations 9 et 10 montrent qu'il est possible d'obtenir directement, par le procédé selon l'invention, des zéolites déjà agglomérées, en utilisant une source de silice et d'alumine déjà agglomérée.

On a utilisé dans ces préparations 9 et 10 une source de silice et d'alumine constituée par une zéolite extrudée du type mordénite.

Pour la préparation N° 9, on a utilisé une zéolite ZEOLON 900H, commercialisée par la Société NORTON, ayant un rapport molaire $SiO_2/Al_2O_3$ de 13.

Pour la préparation N° 10, on a utilisé une zéolite ZEOLON 900Na, commercialisée par la Société NORTON également, préalablement échangée par des ions $H^+$ et désaluminée par un traitement par une solution d'acide chlorhydrique d'une concentration de 12 moles/litre pendant 4 jours, à une température de 90° C, le rapport $SiO_2/Al_2O_3$ étant ainsi égal à 61.

Les compositions molaires des mélanges réactionnels, ramenées à 1 mole de silice contenue dans les zéolites de départ, sont données dans le Tableau X ci-après:

TABLEAU X

| Préparation N° | TPA-Br | $NH_4F$ | $NH_4HF_2$ | $H_2O$ | pH | |
|---|---|---|---|---|---|---|
| | | | | | Initial | Final |
| 9 | 0,2 | 0 | 1,2 | 55 | 3,5 | 6,5 |
| 10 | 0,2 | 0,5 | 0,5 | 55 | 4,5 | 6,5 |

Les deux mélanges réactionnels sont chauffés, dans un autoclave revêtu intérieurement de polytétrafluoroéthylène, à une température de 170° C, pendant 6 jours pour la préparation N° 9 et pendant 4 jours pour la préparation N° 10.

Après séparation par filtration et lavage à l'eau, les extrudés sont relavés avec une solution aqueuse à 30 % de diéthylamine, afin de dissoudre les fluorures complexes d'aluminium et d'ammonium qui peuvent être présents.

Après calcination à une température de 550° C pendant 2 heures et réhydratation, les extrudés sont broyés et examinés par diffraction des rayons X. Il s'agit de zéolites du type ZSM 5 présentant le spectre du Tableau III.

Le produit de la préparation N° 9 contient 2,6 % en poids d'aluminium et celui de la préparation N° 10 1,4 % en poids.

## EXEMPLE 2

Cet exemple concerne la préparation de zéolites par le procédé selon l'invention.

## Préparations N° 11 et N° 12

Les préparations N° 11 et N° 12 montrent que les cations ammonium ou phosphonium quaternaires peuvent être remplacés par les cations tripropylammonium (TRIPA$^+$) ou dipropylammonium (DIPA$^+$). Ces derniers cations sont en effet généralement obtenus de manière plus économique que les premiers.

On utilise un aluminosilicate de sodium (TIXOLEX 28 commercialisé par RHONE POULENC) dont les cations Na$^+$ sont échangés par des cations NH$_4^+$ selon les procédures habituelles. Le rapport molaire SiO$_2$/Al$_2$O$_3$ est égal à 13,3 et la teneur pondérale en Na$_2$O inférieure à 0,02 %. On mélange le TIXOLEX 28 avec une des deux amines, tripropylamine ou dipropylamine, de l'eau et de l'acide chlorhydrique de manière à obtenir deux mélanges réactionnels ayant les caractéristiques données dans le Tableau XI ci-après:

## TABLEAU XI

|  | Préparation N° 11 | Préparation N° 12 |
|---|---|---|
| TIXOLEX 28 * | 1 mole | 1 mole |
| Amine | 1,5 mole de dipropylamine | 1 mole de tripropylamine |
| Eau | 30 moles | 30 moles |
| HF | 2 moles | 1 mole |
| pH | 6,5 | 7 |
| Cristaux de zéolite (germes) | 2,5 % en poids | 2,5 % en poids |

* SiO$_2$ ; 0,075 Al$_2$O$_3$ ; 0,075 (NH$_4$)$_2$O; 0,7 H$_2$O

- 23 -

0172068

Les deux mélanges sont chauffés pendant 7 jours à 170° C dans un autoclave revêtu intérieurement de polytétrafluoroéthylène. Après filtration, lavage à l'eau et calcination à 550° C, on obtient dans les deux préparations des cristaux dont le spectre de diffraction des rayons X est semblable à celui du tableau III. Les cristaux prismatiques ont des dimensions comprises entre 3 et 25 micromètres. Les rapports Si/Al sont respectivement égaux à 24 et 18 pour les préparations N° 11 et N° 12.

EXEMPLE 3

Cet exemple concerne des essais d'adsorption de xylènes à l'aide des zéolites obtenues dans les préparations N° 5 et N° 6 de l'Exemple 1.

Les essais ont été effectués, à l'aide des zéolites activées à 550° C pendant 14 heures, dans une enceinte à pression de vapeur d'adsorbat contrôlée.

Les résultats figurent dans le Tableau XII ci-après:

TABLEAU XII

| Adsorbat | Pression de l'adsorbat en mm de mercure | % pondéral adsorbé à 20° C après 2 heures | |
|---|---|---|---|
| | | Zéolite selon préparation N° | |
| | | 5 | 6 |
| Ortho-xylène | 3,5 | 0,5 | 0,65 |
| Para-xylène | 4 | 12,5 | 12,9 |

Cet exemple permet de constater que l'on peut séparer, à l'aide des zéolites préparées par le procédé selon l'invention, les xylènes entre eux.

EXEMPLE 4

Cet exemple concerne des essais de transformation du méthanol en hydrocarbures à l'aide des zéolites obtenues dans les préparations N° 4 et N° 8.

On a utilisé les zéolites:

- de la préparation N° 4, non traitée à l'ammoniaque, ci-après appelée zéolite Z40,
- de la préparation N° 4, traitée à l'ammoniaque, ci-après appelée zéolite Z41,

- de la préparation N° 8, ci-après appelée zéolite Z8.

Avant les tests catalytiques, les zéolites sont, de façon classique, pour l'utilisation des zéolites comme catalyseurs, mélangées avec du kaolin, à raison de 80 % en poids de zéolite pour 20 % en poids de kaolin dans le mélange.

Les tests sont effectués de la façon suivante:

On place dans un réacteur 1,5 gramme de catalyseur (zéolite + kaolin). On fait passer un mélange d'azote et de méthanol sur le catalyseur, à une température de 370° C à une pression de 1 bar.

La vitesse spatiale horaire (masse de méthanol par masse de catalyseur et par heure) est de 1,1 $h^{-1}$.

On effectue l'analyse de l'effluent sortant du réacteur au bout de différents temps, par chromatographie en phase gazeuse.
Les résultats sont donnés dans le Tableau XIII ci-après:

TABLEAU XIII

| Zéolite du catalyseur | Conversion en hydrocarbures en % poids. au bout de (1) | | | Distribution des hydrocarbures recueillis pendant 60 minutes | | | | | |
|---|---|---|---|---|---|---|---|---|---|
| | 60 minutes | 120 minutes | 180 minutes | %n - P (2) | %i - P (3) | % Ol (4) | % Ar (5) | % C5$^+$ (6) | % Ar dans C5$^+$ (7) |
| Z 40 | 100 | 100 | 100 | 32,43 | 15,25 | 33,31 | 19,00 | 30,47 | 62,35 |
| Z 41 | 100 | 100 | 100 | 19,61 | 16,14 | 21,44 | 42,81 | 52,31 | 81,84 |
| Z 8 | 100 | 100 | 100 | 24,90 | 19,17 | 18,71 | 37,50 | 49,60 | 75,56 |

(1) % de méthanol transformé en hydrocarbures,

(2) % de normal-paraffines dans les hydrocarbures formés,

(3) % d'iso-paraffines dans les hydrocarbures formés,

(4) % d'oléfines dans les hydrocarbures formés,

(5) % d'aromatiques dans les hydrocarbures formés,

(6) % d'hydrocarbures à 5 et plus de 5 atomes de carbone dans les hydrocarbures formés,

(7) % d'hydrocarbures aromatiques dans C5$^+$.

0172068

Le Tableau montre qu'avec les zéolites préparées par le procédé selon l'invention, on obtient une conversion maximum du méthanol en hydrocarbures. De plus, le pourcentage d'hydrocarbures aromatiques formés est élevé, ce qui est très intéressant pour l'utilisation de ces hydrocarbures comme carburant, en raison de l'indice d'octane élevé des hydrocarbures aromatiques.

Revendications

1. Procédé de synthèse de zéolites, ledit procédé consistant:

a) dans une première étape, à former un milieu réactionnel comprenant notamment de l'eau, une source de silice, une source d'alumine, une source d'un agent structurant pouvant fournir des cations choisis dans le groupe constitué par les cations tétrapropylammonium ($TPA^+$), tripropylammonium ($TRIPA^+$), dipropylammonium ($DIPA^+$) et tétrapropylphosphonium ($TPP^+$),

b) dans une deuxième étape, à chauffer le milieu réactionnel formé dans l'étape a) à une température comprise entre 60 et 230° C et, de préférence, entre 80 et 210° C, cette seconde étape conduisant à l'obtention d'un solide cristallisé qui est séparé,

c) dans une troisième étape, à chauffer le solide obtenu dans l'étape b) à une température supérieure à 450° C, ledit procédé étant caractérisé en ce que le milieu réactionnel de l'étape a) contient en outre des anions fluorure $F^-$ et en ce que le pH dudit milieu est inférieur à 10.

2. Procédé selon la revendication 1, caractérisé en ce que le rapport molaire $\frac{fluorure}{silice}$ dans le milieu réactionnel est compris entre 0,04 et 4 et, de préférence, entre 0,06 et 2.

3. Procédé selon l'une des revendications 1 et 2, caractérisé en ce que le rapport molaire $\frac{TPA^+ \text{ ou } TPP^+}{silice}$ dans le milieu réactionnel est compris entre 0,04 et 1 et, de préférence, entre 0,08 et 0,75.

4. Procédé selon l'une des revendications 1 et 2, caractérisé en ce que le rapport molaire $\frac{TRIPA^+}{silice}$ dans le milieu réactionnel est compris entre 0,2 et 2 et, de préférence, entre 0,5 et 1,25.

5. Procédé selon l'une des revendications 1 et 2, caractérisé en ce que le rapport molaire $\underline{DIPA^+}$ dans le milieu réacsilice tionnel est compris entre 0,5 et 2,5 et de préférence entre 0,75 et 2.

6. Procédé selon l'une des revendications 1 à 5, caractérisé en ce que le rapport molaire $\underline{eau}$ dans le milieu réacsilice tionnel est compris entre 3 et 200 et, de préférence, entre 4 et 60.

7. Procédé selon l'une des revendications 1 à 6, caractérisé en ce que le rapport molaire $SiO_2/Al_2O_3$ dans le milieu réactionnel est compris entre 8 et 500 et, de préférence, entre 12 et 400.

8. Procédé selon l'une des revendications 1 à 7, caractérisé en ce que le pH du milieu réactionnel est compris entre 1,5 et 10 et, de préférence, entre 3 et 8,5.

9. Procédé selon l'une des revendications 1 à 8, caractérisé en ce que le milieu réactionnel contient un sel complémentaire et en ce que le rapport molaire $\underline{sel\ complémentaire}$ silice dans le milieu réactionnel est compris entre 0 et 4 et, de préférence, entre 0 et 0,5.

10. Procédé selon l'une des revendications 1 à 9, caractérisé en ce que le milieu réactionnel contient des cristaux de zéolite et en ce que le rapport pondéral $\underline{cristaux\ de\ zéolite}$ silice dans le milieu réactionnel est compris entre 0,01 et 0,10 et, de préférence, entre 0,02 et 0,04.

11. Les zéolites préparées par le procédé selon l'une des revendications 1 à 10.

12. Application des zéolites selon la revendication 11 comme catalyseurs ou supports de catalyseurs.

13. Application des zéolites selon la revendication 11 à la séparation d'hydrocarbures entre eux.

14. Application des zéolites selon la revendication 11 à la conversion de méthanol en hydrocarbures.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | US-A-3 849 463 (F.G. DWYER et al.) <br> * Revendications 3,4,6 et colonne 3, lignes 52-57; colonne 10, tableau 4 et lignes 44-69; colonne 2, lignes 23-25 * | 1,6,7, 9,12 | C 01 B 33/28 <br> B 01 J 29/28 <br> C 07 C 7/13 <br> C 07 C 1/20 |
| | --- | | |
| A | EP-A-0 031 255 (MITSUBISHI GAS CHEMICAL CO.) <br> * Revendication 1; tableau; page 5, revendication 9 et page 8, ligne 20; revendications 14,15,17,18 et tableau, page 11 * | 1-3,6, 7 | |
| A | * Revendication 21, page 12, lignes 10-29 et pages 16,17 * | 12-14 | |
| | --- | | **DOMAINES TECHNIQUES RECHERCHES (Int. Cl.4)** |
| A | EP-A-0 053 499 (MITSUBISHI GAS CHEMICAL CO.) <br> * Revendication 1 et page 4, lignes 18,23; revendications 6,11,12 et tableau 2, pae 6; pae 2, ligne 8 et page 8, ligne 24 * | 1-3,6, 7 | C 01 B 33/28 |
| A | * Page 8, lignes 30-38 et pages 12-14; exemple de référence * | 12,14 | |
| | ---     -/- | | |

Le présent rapport de recherche a été établi pour toutes les revendications

| Lieu de la recherche | Date d'achèvement de la recherche | Examinateur |
|---|---|---|
| LA HAYE | 29-10-1985 | BREBION J.CH. |

Office européen
des brevets

RAPPORT DE RECHERCHE EUROPEENNE

0172068
Numero de la demande

EP  85 40 1415

Page  2

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| L | US-A-4 296 083  (L.D. ROLLMANN)<br>* Exemples 1,2; en particulier colonne 13, lignes 40,41 et 46; colonne 14, lignes 11-13 et 18 *<br>Les 2 exemples montrent qu'en l'absence d'indication de valeur du pH et même en possession de la valeur du rapport molaire OH-/SlO2, il vaut mieux s'abstenir de supputer le pH. Ceci explique l'attribution du code A et non X aux 3 documents précédents.<br><br>--- | 1 | |
| A | EP-A-0 037 983  (BAYER AG)<br><br>* Revendications 1,6 et page 29, exemple 7 *<br><br>--- | | |
| A | FR-A-2 130 430  (MOBIL OIL CORP.)<br>* Exemple 13, pages 25,26 *<br><br>--- | 1 | DOMAINES TECHNIQUES RECHERCHES (Int Cl 4) |
| A | FR-A-2 245 574  (MOBIL OIL CORP.)<br>* Tableau III, page 9, C *<br><br>----- | 1 | |

Le présent rapport de recherche a été établi pour toutes les revendications

| Lieu de la recherche | Date d'achèvement de la recherche | Examinateur |
|---|---|---|
| LA HAYE | 29-10-1985 | BREBION J.CH. |